# EUROPÄISCHE PATENTSCHRIFT

(11) **EP 2 193 745 B1**
(45) Veröffentlichungstag und Bekanntmachung des Hinweises auf die Patenterteilung: **22.06.2011**
(21) Anmeldenummer: 08021124.6
(22) Anmeldetag: 04.12.2008
(51) Int. Cl.: A61B 5/022

(54) **Langzeit-Blutdruckmessgerät**
Long term blood pressure measuring device
Appareil de mesure de la pression sanguine longue durée

(43) Veröffentlichungstag der Anmeldung: 09.06.2010
(73) Patentinhaber: Müller & Sebastiani Elektronik GmbH, 85521 Ottobrunn (DE)
(72) Erfinder: Müller, Peter, 80469 München (DE)
(74) Vertreter: Manitz, Finsterwald & Partner GbR

(56) Entgegenhaltungen:
- EP-A- 0 467 853
- EP-A- 0 542 413
- EP-A- 0 875 200
- WO-A-96/11625

## Beschreibung

Die Erfindung betrifft ein Langzeit-Blutdruckmessgerät mit einem am Körper einer Person anschließbarer Messaufnehmer für die Erfassung des Blutdrucks, einer Auswerteeinheit zur Ermittlung von Blutdruckwerten und einem Signalspeicher zur Speicherung von Blutdruckwerten.

Derartige Geräte sind seit längerer Zeit bekannt und werden z.B. in mobiler Form eingesetzt, um über vergleichsweise lange Zeiträume von mehreren Stunden oder mehreren Tagen am Körper von Patienten getragen zu werden. Bei diesen bekannten Geräten erfolgen Blutdruckmessungen in der Regel periodisch in fest vorgegebenen Zeitabständen, zum Beispiel alle 30 Minuten. Dies führt auf nachteilige Weise dazu, dass signifikante Blutdruckveränderungen dann unerkannt bleiben, wenn sie in den Zeitintervallen zwischen zwei Messungen auftreten. Weiterhin werden bei regelmäßig stattfindenden Messungen auch unnötige Messungen bei unveränderten Blutdruckwerten durchgeführt, wobei diese Messungen die das Gerät tragende Person dann unnötig belasten oder irritieren.

EP 0 875 200 A1 und WO 96/11625 A offenbaren Langzeit-Blutdruckmessgeräte gemäß dem Oberbegriff des Anspruchs 1, bei denen eine Aktivierung einer Blutdruckmessung in Abhängigkeit vom ermittelten Verlauf eines Pulswellensignals erfolgt. Weitere Blutdruckmessgeräte sind in EP 0 467 853 A1 und EP 0 542 414 A1 offenbart.

Eine Aufgabe der vorliegenden Erfindung besteht dementsprechend darin, ein Gerät der eingangs genannten Art weiterzuentwickeln, welches verbesserte Diagnosemöglichkeiten bereitstellt.

Erfindungsgemäß wird diese Aufgabe durch die Merkmale des Anspruchs 1 gelöst.
Es ist ein zusätzlicher, am Körper einer Person anschließbarer Messaufnehmer für den Verlauf eines Pulswellensignals vorgesehen und der Signalspeicher ist zur zusätzlichen Speicherung dieses Verlaufs ausgelegt, wobei weiterhin eine Einrichtung zur Aktivierung einer Blutdruckmessung vorgesehen ist, die zur Abgabe eines Aktivierungssignals in Abhängigkeit vom ermittelten Verlauf des Pulswellensignals ausgelegt ist.

Mit dem zusätzlichen Messaufnehmer wird - insbesondere ständig - das Pulswellensignal einer jeweiligen Person erfasst und ausgewertet. Ein Pulswellensignal zeigt - z.B. im Unterschied zu Körperstrom- bzw. EKG-Signalen - zumindest qualitativ an, wie groß der Blutstrom einer Person im Bereich ihrer Extremitäten zu einem bestimmten Zeitpunkt ist, so dass man sich die Erkenntnis zu Nutze macht, dass das Auftreten von Veränderungen von Blutdruckwerten einen Niederschlag im Verlauf des Pulswellensignals findet. Dementsprechend kann dann ein veränderter Verlauf des Pulswellensignals zur gezielten Aktivierung einer Blutdruckmessung zu solchen Zeitpunkten führen, zu denen veränderte Blutdruckwerte auftreten.

Zu diesem Zweck kann das Pulswellensignal auf unterschiedliche Art und Weise ausgewertet werden. Besonders vorteilhaft ist es dabei, wenn eine Einrichtung zur Aktivierung einer Blutdruckmessung vorgesehen ist, welche den zeitlichen Abstand t₁ zweier benachbarter Maxima einzelner Pulswellen-Impulse, insbesondere den zeitlichen Abstand t₁ zwischen Hauptmaximum und größtem Nebenmaximum einzelner Pulswellen-Impulse detektiert, wobei die Einrichtung zur Aktivierung einer Blutdruckmessung zur Abgabe eines Aktivierungssignals bei kleiner oder größer werdendem zeitlichen Abstand t₁ ausgelegt ist.
Hierbei wird die Erkenntnis genutzt, dass bei einem kleiner werdenden zeitlichen Abstand t₁ der Blutdruck in der Regel steigt, wohingegen er bei einem größer werdenden zeitlichen Abstand t₁ fällt.

Bevorzugt ist es, wenn die Korrelation zwischen den gemessenen Blutdruckwerten und den zum jeweiligen Messzeitpunkt gegebenen zeitlichen Abständen t₁ ermittelt wird, wobei bei Überschreiten eines vorgegebenen Grenzwerts durch den ermittelten Korrelationswert weitere Blutdruckwerte aus den zwischen den Blutdruckmessungen erfassten zeitlichen Abständen t₁ berechnet werden. Anhand des Korrelationswerts kann in diesem Fall bestimmt werden, ob bei einem bestimmten Patienten nicht nur ein qualitativer, sondern auch ein quantitativer Zusammenhang zwischen den ermittelten Abständen t₁ und den gemessenen Blutdruckwerten existiert. Wenn dies der Fall ist, was sich durch einen ausreichend großen Korrelationswert ausdrückt, kann ein rechnerischer Bezug zwischen den ermittelten Abständen t₁ und den gemessenen Blutdruckwerten hergestellt werden, der im einfachsten Fall einem Proportionalbezug entspricht. Dementsprechend können dann auch aus solchen Abständen t₁ Blutdruckwerte berechnet werden, die zwischen tatsächlich erfolgten Blutdruckmessungen ermittelt wurden. Es lassen sich also rein rechnerisch Blutdruckwerte zu Zeitpunkten bestimmen, zu denen keine physikalischen Blutdruckmessungen mit einer Druckmanschette erfolgt sind, was sich hinsichtlich der beim Patienten auftretenden Belastungen sehr positiv auswirkt.

Das erfindungsgemäße Gerät kann derart betrieben werden, dass Blutdruckmessungen nur dann ausgeführt werden, wenn der zeitliche Abstand t₁ kleiner wird. Auf diese Weise werden Blutdruckwerte nur dann ermittelt, wenn - was aus medizinischer Sicht besonders interessant ist - der Blutdruck steigt. Unnötige Messungen, die den Patienten zusätzlich belasten, werden dabei vollständig vermieden. Ebenso ist es jedoch auch möglich, Blutdruckmessungen sowohl bei kleiner als auch bei größer werdendem zeitlichen Abstand t₁ durchzuführen, so dass zusätzlich auch dann Blutdruckmessungen durchgeführt werden, wenn der Blutdruck fällt. Weiterhin ist es möglich, zusätzlich zu den durch das Pulswellensignal ausgelösten Blutdruckmessungen auch noch periodische Blutdruckmessungen in fest vorgegebenen Zeitabständen durchzuführen, wenn dies medizinisch von Interesse sein sollte.

Es ist weiterhin bevorzugt, wenn zumindest ein am Körper einer Person anschließbarer Messaufnehmer für Körperstromsignale vorgesehen und der Signalspeicher zur Speicherung des Verlaufs der Körperstromsignale ausgelegt ist. Mit einem solchen Gerät können zusätzlich zu Blutdruckmesswerten und Pulswellensignalen auch Körperstrom- bzw. EKG-Signale erfasst werden, welche Aufschluss darüber liefern, zu welchem Zeitpunkt und in welcher Weise der Herzmuskel der jeweiligen Person erregt wurde. Auf Basis derartiger Signale lassen sich dann durch manuelle oder automatische Auswertungen solche Störungen in der Herzerregung diagnostizieren, die im Rahmen einer ambulanten, auf einen vergleichsweise kurzen Zeitraum beschränkten Untersuchung nicht ermittelt werden könnten.

Wenn das erfindungsgemäße Gerät Messaufnehmer für Körperstromsignale aufweist, ist es besonders vorteilhaft, eine Einrichtung zur Ermittlung der Pulswellenlaufzeit aus dem zeitlichen Bezug zwischen den Verläufen von Körperstromsignalen und Pulswellensignal vorzusehen. Die Pulswellenlaufzeit kann dabei dem zeitlichen Abstand zwischen nacheinander auftretenden Maxima in den Verläufen von Körperstromsignalen und Pulswellensignal entsprechen.

Die Pulswellenlaufzeit kann dabei zu bestimmten Zeitpunkten, alternativ dazu jedoch auch kontinuierlich ermittelt werden, wobei die ermittelten Werte gemeinsam mit ihrem zeitlichen Bezug innerhalb des erfindungsgemäßen Geräts speicherbar sind. Anstelle von Einzelimpulsen des Körperstrom- und des Pulswellensignals können auch zuerst mehrere (z.B. 5 - 30), zeitlich aufeinander folgende Einzelimpulse gemittelt und dann jeweils der gemittelte Einzelimpulsverlauf zur Bestimmung der Pulswellenlaufzeit ausgewertet werden.

Letztlich zeigt die Pulswellenlaufzeit an, wie lange es dauert, bis eine von einem Herzschlag ausgelöste Pulswelle in den Extremitäten einer Person ankommt. Dabei kann davon ausgegangen werden, dass die Pulswellenlaufzeit sich umgekehrt zum Blutdruck verhält, was bedeutet, dass ein erhöhter Blutdruck eine verkürzte Pulswellenlaufzeit nach sich zieht.

Bei Ermittlung der Pulswellenlaufzeit kann die Auswerteeinheit des erfindungsgemäßen Geräts in einer Art und Weise betrieben werden, dass sie zur Detektion einer gegenüber bereits ermittelten Werten verkürzten oder verlängerten Pulswellenlaufzeit geeignet ist, so dass dann infolge einer solchen Detektion eine Blutdruckmessung ausgelöst werden kann.

Es existieren also zwei unterschiedliche Möglichkeiten zur Aktivierung einer Blutdruckmessung, die alternativ oder auch gemeinsam zum Einsatz gelangen können. Zum einen kann die Aktivierung der Blutdruckmessung in Abhängigkeit vom zeitlichen Abstand t₁ zweier benachbarter Maxima einzelner Pulswellen-Impulse erfolgen. Zum anderen ist auch eine vorstehend erwähnte Aktivierung in Abhängigkeit von der Pulswellenlaufzeit möglich. Darüber hinaus kann natürlich in jedem Fall eine periodische Aktivierung von Blutdruckmessungen erfolgen, wenn dies zusätzlich gewünscht ist.

Wenn ein Pulswellensignal ermittelt wird, ergeben sich eine Reihe von weiteren Diagnosemöglichkeiten:

Durch die Ermittlung des Verlaufs des Pulswellensignals wird es möglich, für jede einzelne Herzerregung zu ermitteln, welche Wirkung bzw. welche Effizienz ein durch die Erregung ausgelöster Herzschlag besitzt, was bisher bei mobilen EKG-Geräten nicht möglich war. Es lässt sich also beispielsweise feststellen, wenn eine weitgehend normale Herzerregung zu einem unauffälligen Körperstromsignal führt, in der Folge diese Herzerregung aber keine ausreichende Wirkung im Pulswellensignalverlauf zeigt. Ein solcher Fall lässt sich mit bisher üblichen EKG-Geräten nicht ermitteln, so dass hier neue und erweiterte Diagnosemöglichkeiten zur Verfügung gestellt werden.
Weiterhin lässt sich beispielsweise auch feststellen, wenn bei weitgehend normaler Herzerregung die Effizienz der Herzschläge langsam abnimmt, da ein solches langsames Abnehmen sich in veränderten Pulswellensigrialformen bzw. in einem veränderten zeitlichen Verlauf des Pulswellensignals niederschlägt. Rein beispielhaft sei angeführt, dass ein Abnehmen der Effizienz der Herzschläge zu einer mit der Zeit verringerten Amplitude des Pulswellensignals führen kann.

Die im Gerät vorhandene Auswerteeinheit ist erfindungsgemäß so ausgelegt, dass sie während des Aufblasens oder des Entlüftens einer am Arm eines Patienten befindlichen Druckmanschette den systolischen Blutdruck zu demjenigen Zeitpunkt ermittelt, zu dem die Amplitude des Pulswellensignals während einer Periode dieses Pulswellensignals unter einen vorgegebenen Schwellwert absinkt bzw. über einen solchen Schwellwert ansteigt.

So kann z.B. exakt derjenige Zeitpunkt bestimmt werden, zu dem eine am Arm eines Patienten befindliche Druckmanschette einen so großen Druck aufbaut, dass in der jeweiligen Extremität kein Blut mehr fließt, so dass das Pulswellensignal zumindest weitgehend gleich Null wird, auf jeden Fall aber während einer Periode des Pulswellensignals unter einen vorgegebenen Schwellwert absinkt. Der in der Druckmanschette zu diesem Zeitpunkt herrschende Druck ist dann der systolische Blutdruck. Alternativ ist es natürlich auch möglich, denjenigen Zeitpunkt zu bestimmen, zu dem der Druck einer am Arm eines Patienten befindlichen Druckmanschette so weit verringert wurde, dass in der jeweiligen Extremität das Blut wieder beginnt zu fließen, so dass das Pulswellensignal während einer Periode des Pulswellensignals über einen vorgegebenen Schwellwert ansteigt. Auch der in der Druckmanschette zu diesem Zeitpunkt herrschende Druck entspricht wiederum dem systolischen Blutdruck.
Dieses Verfahren ermöglicht somit eine sehr exakte Bestimmung des systolischen Blutdrucks.

Die Auswerteeinheit kann dann weiter zur aus dem Stand der Technik bekannten oszillometrischen Ermittlung des diastolischen Blutdrucks ausgelegt sein.

Der Messaufnehmer zur Erfassung des Pulswellensignals kann in üblicher Weise aus zumindest einer Strahlungs-, insbesondere einer Lichtquelle und zumindest einem Empfangselement, insbesondere einer Fotodiode bestehen, wobei der Messaufnehmer vorzugsweise an einem Finger oder an einem Ohrläppchen einer Person angeschlossen werden kann. Alternative Ausbildungen des zusätzlichen Messaufnehmers sind jedoch ebenso möglich.

Besonders bevorzugt ist es, wenn der zusätzliche Messaufnehmer nicht nur zur Erfassung des Pulswellensignals sondern zudem auch zur Ermittlung des Verlaufs des Sauerstoffpartialdrucks (pO₂) einer Person ausgelegt ist. In diesem Fall ist es dann sinnvoll, den im Gerät vorhandenen Signalspeicher so auszulegen, dass er zusätzlich auch den Verlauf des Sauerstoffpartialdrucks speichern kann.

Die Auswertung der erfindungsgemäß aufgenommenen Signale (Körperstromsignale, Verlauf des Pulswellensignals und gegebenenfalls Verlauf des Sauerstoffpartialdrucks) kann mittels beliebiger Auswerteeinheiten vorgenommen werden. Eine solche Auswerteeinheit kann beispielsweise in ein erfindungsgemäßes tragbares Gerät integriert werden. Ebenso ist es jedoch möglich, eine externe Auswerteeinheit, beispielsweise in Form eines PCs oder eines Laptops, vorzusehen, die auf die erfindungsgemäß gespeicherten Daten zugreift. Insbesondere ist es möglich, eine erste Auswerteeinheit innerhalb des tragbaren Geräts anzuordnen, die eine erste grobe Diagnose erstellt und dabei besonders interessante Signalabschnitte ermittelt, wobei dann eine aufwändigere externe Auswerteeinheit die zuvor selektierten Signalabschnitte oder aber auch die kompletten Signale genauer auswertet.

Eine erfindungsgemäße Auswerteeinheit ist insbesondere zur Detektion von Unregelmäßigkeiten des Verlaufs des Pulswellensignals, insbesondere zur Detektion von veränderten Pulswellensignalformen oder eines veränderten zeitlichen Verlaufs des Pulswellensignals oder eines Amplitudenabfalls des Pulswellensignals gegenüber bereits ermittelten Werten und zur Auswertung des Verlaufs der Körperstromsignale in demjenigen Zeitabschnitt ausgelegt, indem eine Unregelmäßigkeit festgestellt wurde. Wie bereits erläutert, kann z.B. ein solcher Amplitudenabfall eine pathologisch verringerte Effizienz des Herzschlags anzeigen, so dass in einem solchen Falle untersucht werden kann, ob diese verringerte Effizienz auf eine eventuelle fehlerhafte Erregung des Herzmuskels zurückzuführen ist.

Wenn das Pulswellensignal in der Auswerteeinheit verarbeitet wird, können dessen Einzelimpulse betrachtet werden. Vorteilhaft ist aber, wenn in der Auswerteeinheit eine Vorrichtung zur Mittelung des Verlaufs des Pulswellensignals vorgesehen ist, die jeweils den Beginn der für eine Mittelung heranzuziehenden Einzelimpulse aufgrund des zeitlichen Bezugs zu den jeweils zugehörigen Körperstromsignalen festlegt. Es wird also zum Beispiel für alle im Rahmen einer Mittelung zu berücksichtigenden Einzelimpulse zuerst ein zeitlicher Bezugspunkt im Körperstromsignal festgelegt, wobei als Bezugspunkt z.B. das Maximum desjenigen Einzelimpulses des Körperstromsignals verwendet wird, der unmittelbar vor dem jeweiligen Einzelimpuls des Pulswellensignals aufgetreten ist. Anschließend wird dann definiert, dass der jeweilige Einzelimpuls des Pulswellensignals in bestimmten zeitlichen Abständen vom Bezugspunkt beginnt und endet. Diese Abstände werden dann für alle folgenden Paare von Einzelimpulsen des Pulswellen- und Körperstromsignals, die für die jeweilige Mittelung maßgeblich sind, ebenfalls herangezogen, so dass es auf diese Weise möglich wird, alle zu mittelnden Einzelimpulse des Pulswellensignals ohne gegenseitige Phasenverschiebung zu mitteln.

Besonders vorteilhaft ist es, wenn die Auswerteeinheit zur Detektion von Herzschrittmachersignalen und zur Auswertung des Verlaufs des Pulswellensignals in demjenigen Zeitabschnitt ausgelegt ist, in dem Herzschrittmachersignale festgestellt wurden. Herzschrittmachersignale sind dabei im Verlauf der Körperstromsignale in der Regel gut feststellbar, da sie normalerweise zu unnatürlichen, problemlos detektierbaren Amplitudenspitzen führen. Somit wird es erfindungsgemäß möglich, zu überprüfen, welche Effizienz jeder durch einen Herzschrittmacher ausgelöste Herzschlag besitzt, so dass über einen langen Zeitraum die Effizienz von Herzschrittmachern gezielt in denjenigen Zeitabschnitten überwacht werden kann, in denen ein Herzschrittmacher jeweils aktiv ist.

Die Erfindung betrifft auch ein Verfahren zum Betrieb eines Langzeit-Blutdruckmeßgeräts gemäß den vorstehend beschriebenen Varianten.

Weitere bevorzugte Ausführungsformen der Erfindung sind in den Unteransprüchen angegeben.

Die Erfindung wird nachfolgend anhand eines Ausführungsbeispiels unter Bezugnahme auf die Zeichnungen erläutert; in diesen zeigen:
- Fig. 1: ein vereinfachtes Blockschaltbild eines erfindungsge- mäßen mobilen Langzeit-Blutdruckmeßgerät,
- Fig. 2a, b: Verläufe von erfindungsgemäß aufgezeichneten Kör- perstrom- und Pulswellensignalen, und
- Fig. 3: den qualitativen Verlauf eines weiteren Pulswellensig- nals.

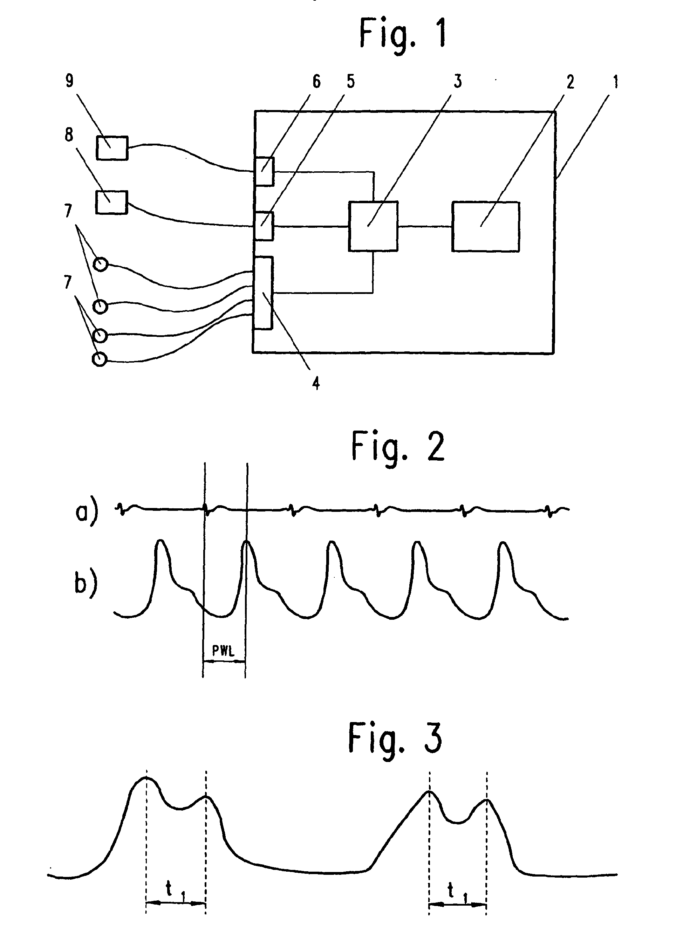

Das Blockschaltbild gemäß Fig. 1 zeigt ein Gehäuse 1, welches am Körper einer nicht dargestellten Person getragen werden kann. Im Gehäuse 1 ist ein mit einem Signalspeicher 2 verbundener Mikroprozessor 3 angeordnet, wobei der Mikroprozessor 3 mit insgesamt drei unterschiedlichen Schnittstellen 4, 5 und 6 in Verbindung steht.

An der Schnittstelle 4 sind herkömmliche Elektroden 7 für die Aufnahme von Körperstrom- bzw. EKG-Signalen anschließbar. Die Schnittstelle 5 ist mit einem Messaufnehmer 8 für den Verlauf eines Pulswellensignals gekoppelt. An der Schnittstelle 6 ist ein Messaufnehmer 9 für die Erfassung von Blutdruckwerten angeschlossen.

Die Vorrichtung gemäß Fig. 1 kann in einer Weise betrieben werden, wie sie vorstehend erläutert wurde.

Fig. 2a zeigt einen typischen zeitlichen Verlauf eines EKG-Signals, welches beispielsweise mit Elektroden 7 aufgenommen, über die Schnittstelle 4 dem Mikroprozessor 3 zugeführt und anschließend im Speicher 2 abgelegt wurde.

In entsprechender Weise zeigt Fig. 2b den zeitlichen Verlauf eines Pulswellensignals, welches mit einem Messaufnehmer 8 abgegriffen wurde.

Der zeitliche Abstand zwischen nacheinander auftretenden Maxima in den Verläufen des EKG-Signals gemäß Fig. 2a und des Pulswellensignals gemäß Fig. 2b entspricht der vorstehend bereits erläuterten Pulswellenlaufzeit. Eine Verkürzung dieser Pulswellenlaufzeit kann, wie bereits erläutert, beispielsweise dazu führen, dass der Mikroprozessor 3 eine Blutdruckmessung über den Messaufnehmer 9 veranlasst.

Fig. 3 zeigt lediglich qualitativ einen alternativen Verlauf eines Pulswellensignals, wobei hier jeder der beiden dargestellten Impulse dieses Pulswellensignals zwei deutliche Maxima, nämlich ein Hauptmaximum und ein Nebenmaximum aufweist. Zur besseren Erkennbarkeit des erfindungsgemäßen Prinzips ist die Ausprägung der Nebenmaxima in Fig. 3 gegenüber realen Signalen etwas verstärkt. Erfindungsgemäß wird der zeitliche Abstand t₁ zwischen den dargestellten Hauptmaxima und Nebenmaxima eines jeden Pulswellen-Impulses bestimmt, wobei die Aktivierung einer Blutdruckmessung beispielsweise dann ausgelöst werden kann, wenn festgestellt wird, dass sich der genannte zeitliche Abstand t₁ verringert oder vergrößert.

### Bezugszeichenliste

- 1: Gehäuse
- 2: Signalspeicher
- 3: Mikroprozessor
- 4: Schnittstelle
- 5: Schnittstelle
- 6: Schnittstelle
- 7: Elektroden
- 8: Messaufnehmer
- 9: Messaufnehmer

## Patentansprüche

1. Langzeit-Blutdruckmessgerät mit einem am Körper einer Person anschließbaren Messaufnehmer (9) für die Erfassung des Blutdrucks, einer Auswerteeinheit (3) zur Ermittlung von Blutdruckwerten und einem Signalspeicher (2) zur Speicherung von Blutdruckwerten,
wobei ein zusätzlicher, am Körper einer Person anschließbarer Messaufnehmer (8) für den Verlauf eines Pulswellensignals vorgesehen ist und der Signalspeicher (2) zur zusätzlichen Speicherung dieses Verlaufs ausgelegt ist, und wobei
eine Einrichtung (3) zur Aktivierung einer Blutdruckmessung vorgesehen ist, die zur Abgabe eines Aktivierungssignals in Abhängigkeit vom ermittelten Verlauf des Pulswellensignals ausgelegt ist,
**dadurch gekennzeichnet,**
**dass** die Auswerteeinheit (3) zur Ermittlung des systolischen Blutdrucks zu demjenigen Zeitpunkt ausgelegt ist, zu dem die Amplitude des Pulswellensignals während einer Periode dieses Signals unter einen vorgegebenen Schwellwert abfällt oder über einen vorgegebnen Schwellwert ansteigt.

2. Langzeit-Blutdruckmessgerät nach Anspruch 1,
**dadurch gekennzeichnet,**
**dass** eine Einrichtung (3) zur Detektion des zeitlichen Abstands t₁ zweier benachbarter Maxima einzelner Pulswellen-Impulse, insbesondere des zeitlichen Abstands t₁ zwischen Hauptmaximum und größtem Nebenmaximum einzelner Pulswellen-Impulse vorgesehen ist, wobei die Einrichtung (3) zur Aktivierung einer Blutdruckmessung zur Abgabe eines Aktivierungssignals bei kleiner oder größer werdendem zeitlichen Abstand t₁ ausgelegt ist.

3. Langzeit-Blutdruckmessgerät nach einem der vorhergehenden Ansprüche,
**dadurch gekennzeichnet,**
**dass** zumindest ein am Körper einer Person anschließbarer Messaufnehmer (7) für Körperstromsignale vorgesehen und der Signalspeicher (2) zur Speicherung des Verlaufs der Körperstromsignale ausgelegt ist.

4. Langzeit-Blutdruckmessgerät nach Anspruch 3,
**dadurch gekennzeichnet,**
**dass** eine Einrichtung (3) zur Ermittlung der Pulswellenlaufzeit (PWL) aus dem zeitlichen Bezug zwischen den Verläufen von Körperstromsignalen und Pulswellensignal vorgesehen ist.

5. Langzeit-Blutdruckmessgerät nach Anspruch 4,
**dadurch gekennzeichnet,**
**dass** die Pulswellenlaufzeit dem zeitlichen Abstand zwischen nacheinander auftretenden Maxima in den Verläufen von Körperstromsignalen und Pulswellensignal entspricht.

6. Langzeit-Blutdruckmessgerät nach einem der Ansprüche 4 oder 5,
**dadurch gekennzeichnet,**
**dass** die Einrichtung (3) zur Aktivierung einer Blutdruckmessung zur Abgabe eines Aktivierungssignals bei Detektion einer gegenüber bereits ermittelten Werten verkürzten oder verlängerten Pulswellenlaufzeit ausgelegt ist.

7. Langzeit-Blutdruckmessgerät nach einem der vorhergehenden Ansprüche,
**dadurch gekennzeichnet,**
**dass** die Auswerteeinheit (3) zur oszillometrischen Ermittlung des diastolischen Blutdrucks ausgelegt ist.

8. Langzeit-Blutdruckmessgerät nach einem der vorhergehenden Ansprüche,
**dadurch gekennzeichnet,**
**dass** der Messaufnehmer (8) für den Verlauf eines Pulswellensignals eine Strahlungs-, insbesondere eine Lichtquelle und ein Empfangselement, insbesondere eine Photodiode aufweist und vorzugsweise an einem Finger oder an einem Ohrläppchen einer Person anschließbar ist.

9. Langzeit-Blutdruckmessgerät nach einem der vorhergehenden Ansprüche,
**dadurch gekennzeichnet,**
**dass** der Messaufnehmer (8) für den Verlauf eines Pulswellensignals zur Ermittlung des Verlaufs des Sauerstoffpartialdrucks (pO₂) einer Person ausgelegt ist und dass der Signalspeicher (2) zur zusätzlichen Speicherung dieses Verlaufs ausgelegt ist.

10. Langzeit-Blutdruckmessgerät nach einem der vorhergehenden Ansprüche,
**dadurch gekennzeichnet,**
**dass** die Auswerteeinheit (3) zur Detektion von Unregelmäßigkeiten des Verlaufs des Pulswellensignals, insbesondere zur Detektion von veränderten Pulswellensignalformen oder eines veränderten zeitlichen Verlaufs des Pulswellensignals oder eines Amplitudenabfalls des Pulswellensignals gegenüber bereits ermittelten Werten, und zur Auswertung des Verlaufs der Körperstromsignale in demjenigen Zeitabschnitt ausgelegt ist, in dem eine Unregelmäßigkeit festgestellt wurde.

11. Langzeit-Blutdruckmessgerät nach einem der vorhergehenden Ansprüche,
**dadurch gekennzeichnet,**
**dass** in der Auswerteeinheit (3) eine Vorrichtung zur Mittelung des Verlaufs des Pulswellensignals vorgesehen ist, die jeweils den Beginn der für eine Mittelung heranzuziehenden Einzelimpulse aufgrund des zeitlichen Bezugs zu den jeweils zugehörigen Körperstromsignalen festlegt.

12. Langzeit-Blutdruckmessgerät nach einem der Ansprüche 3-6,
**dadurch gekennzeichnet,**
**dass** die Auswerteeinheit (3) zur Detektion von Herzschrittmachersignalen, insbesondere von unnatürlichen Amplitudenspitzen, im Verlauf der Körperstromsignale und zur Auswertung des Verlaufs des Pulswellensignals in demjenigen Zeitabschnitt ausgelegt ist, in dem Herzschrittmachersignale festgestellt wurden.

13. Langzeit-Blutdruckmessgerät nach einem der vorhergehenden Ansprüche,
**dadurch gekennzeichnet,**
**dass** es als mobiles Gerät mit einem am Körper einer Person tragbaren, mit einer Energiequelle ausgestatteten Gehäuse (1) ausgebildet ist, und/oder
**dass** die Auswerteeinheit (3) im Gehäuse (1) vorgesehen oder als externe Einheit ausgebildet ist.

14. Langzeit-Blutdruckmessgerät nach einem der Ansprüche 2 bis 13,
**dadurch gekennzeichnet,**
**dass** die Korrelation zwischen den gemessenen Blutdruckwerten und den zum jeweiligen Messzeitpunkt gegebenen zeitlichen Abständen t₁ gemäß Anspruch 2 ermittelt wird, wobei bei Überschreiten eines vorgegebenen Grenzwerts durch den ermittelten Korrelationswert weitere Blutdruckwerte aus den zwischen den Blutdruckmessungen erfassten zeitlichen Abständen t₁ gemäß Anspruch 2 berechnet werden.

## Claims

1. A long-term blood pressure measuring device having a measurement sensor (9) attachable to the body of a person for detecting the blood pressure, having an evaluation unit (3) for determining blood pressure values and having a signal memory (2) for storing blood pressure values,
wherein an additional measuring sensor (8) attachable to the body of a person is provided for the characteristic of a pulse wave signal and the signal memory (2) is designed for the additional storage of this characteristic; and wherein
a device (3) is provided for activating a blood pressure measurement and is designed to output an activation signal in dependence on the determined characteristic of the pulse wave signal, **characterised in that**
the evaluation unit (3) is designed for determining the systolic blood pressure at that point in time at which the amplitude of the pulse wave signal drops below a preset threshold value or increases above a preset threshold value during a period of this signal.

2. A long-term blood pressure measuring device in accordance with claim 1,
**characterised in that**
a device (3() is provided for detecting the time interval t₁ of two adjacent maxima of individual pulse wave impulses, in particular the time interval t₁ between the main maximum and the largest secondary maximum of individual pulse wave impulses, with the device (3) being designed for activating a blood pressure measurement for outputting an activation signal on a time interval t₁ becoming smaller or larger.

3. A long-term blood pressure measuring device in accordance with one of the preceding claims;
**characterised in that**
at least measurement sensor (7) attachable to the body of a person for body current signals is provided and the signal memory (2) is designed for storing the characteristic of the body current signal.

4. A long-term blood pressure measuring device in accordance with claim 3,
**characterised in that**
a device (3) is provided for determining the pulse wave transit time (PWTT) from the time relationship between the characteristics of body current signals and the pulse wave signal.

5. A long-term blood pressure measuring device in accordance with claim 4,
**characterised in that**
the pulse wave transit time corresponds to the time interval between sequentially occurring maxima in the characteristics of body current signals and the pulse wave signal.

6. A long-term blood pressure measuring device in accordance with one of the claims 4 or 5,
**characterised in that**
the device (3) for activating a blood pressure measurement for outputting an activation signal on detection of a pulse wave transit time shortened or lengthened with respect to already determined values.

7. A long-term blood pressure measuring device in accordance with any one of the preceding claims.
**characterised in that**
the evaluation unit (3) is designed for the oscillometric determination of the diastolic blood pressure.

8. A long-term blood pressure measuring device in accordance with any one of the preceding claims.
**characterised in that**
the measurement sensor (8) for the characteristic of a pulse wave signal) has a radiation source, in particular a light source, and a reception element, in particular a photodiode, and is preferably attachable to a finger or to an earlobe of a person.

9. A long-term blood pressure measuring device in accordance with any one of the preceding claims.
**characterised in that**
the measurement sensor (8) for the characteristic of a pulse wave signal is designed for determining the characteristic of the oxygen partial pressure (pO₂) of a person; and **in that** the signal memory (2 is designed for the additional storing of this characteristic.

10. A long-term blood pressure measuring device in accordance with any one of the preceding claims.
**characterised in that**
the evaluation unit (3) is designed for detecting irregularities of the characteristic of the pulse wave signal, in particular for detecting changed pulse wave signal shapes or of a changed time characteristic of the pulse wave signal or of an amplitude drop of the pulse wave signal with respect to already determined values and for evaluating the characteristic of the body current signals **in that** time portion in which an irregularity was determined.

11. A long-term blood pressure measuring device in accordance with any one of the preceding claims.
**characterised in that**
an apparatus is provided in the evaluation unit (3) for averaging the characteristic of the pulse wave signal, said apparatus in each case fixing the start of the individual impulses to be used for an averaging on the basis of the time relationship with respect to the respective associated body current signals.

12. A long-term blood pressure measuring device in accordance with any one of the claims 3 to 6.
**characterised in that**
the evaluation unit (3) is designed for detecting pacemaker signals, in particular unnatural amplitude peaks, in the characteristic of the body current signals and for evaluating the characteristic of the pulse wave signal **in that** time portion in which pacemaker signals were found.

13. A long-term blood pressure measuring device in accordance with any one of the preceding claims.
**characterised in that**
it is formed as a mobile device having a housing (1) portable on the body of a person and equipped with an energy source; and/or **in that** the evaluation unit (3) is provided in the housing (1) or is formed as an external unit.

14. A long-term blood pressure measuring device in accordance with any one of the claims 2 to 13,
**characterised in that**
the correlation between the measured blood pressure values and the time intervals t₁ present at the respective measurement time is determined in accordance with claim 2, wherein on the exceeding of a preset limit value by the determined correlation value further blood pressure values are calculated from the time intervals t₁ detected between the blood pressure measurements in accordance with claim 2.

## Revendications

1. Appareil de mesure de la pression sanguine sur une longue durée, comprenant un capteur de mesure (9) à appliquer sur le corps d'une personne pour la détection de la pression sanguine, une unité d'évaluation (3) pour déterminer les valeurs de pression sanguine, et une mémoire de signaux (2) pour mémoriser des valeurs de pression sanguine,
dans lequel il est prévu un capteur de mesure supplémentaire (8), à appliquer sur le corps d'une personne, pour l'évolution d'un signal ondulatoire à impulsions, et la mémoire de signaux (2) est conçue pour mémoriser additionnellement cette évolution, et dans lequel il est prévu un moyen (3) pour activer une mesure de la pression sanguine, qui est conçu pour fournir un signal d'activation en fonction de l'évolution déterminée du signal ondulatoire à impulsions,
**caractérisé en ce que** l'unité d'évaluation (3) est conçu pour déterminer la pression sanguine de systole à l'instant auquel l'amplitude du signal ondulatoire à impulsions chute pendant une période de ce signal au-dessous d'une valeur seuil prédéterminée ou monte au-dessus d'une valeur seuil prédéterminée.

2. Appareil de mesure de la pression sanguine sur une longue durée selon la revendication 1,
**caractérisé en ce qu'**il est prévu un moyen (3) pour la détection de la distance temporelle t₁ de deux maxima voisins d'impulsions ondulatoires individuelles, en particulier de la distance temporelle t₁ entre un maximum principal et le plus grand maximum annexe d'impulsions ondulatoires individuelles, ledit moyen (3) étant conçu pour l'activation d'une mesure de pression sanguine pour fournir un signal d'activation lorsque la distance temporelle t₁ devient plus petite ou plus grande.

3. Appareil de mesure de la pression sanguine sur une longue durée selon l'une des revendications précédentes,
**caractérisé en ce qu'**il est prévu au moins un capteur de mesure (7) à appliquer sur le corps d'une personne, pour des signaux électriques corporels, et la mémoire de signaux (2) est conçue pour mémoriser l'évolution des signaux électriques corporels.

4. Appareil de mesure de la pression sanguine sur une longue durée selon la revendication 3,
**caractérisé en ce qu'**il est prévu un moyen (3) pour déterminer le temps de parcours des ondulations à impulsions (PWL) à partir de la relation temporelle entre les évolutions des signaux électriques corporels et du signal ondulatoire à impulsions.

5. Appareil de mesure de la pression sanguine sur une longue durée selon la revendication 4, **caractérisé en ce que** le temps de parcours des ondulations à impulsions correspond à l'écart temporel entre des maxima apparaissant successivement dans les évolutions des signaux électriques corporels et du signal ondulatoire à impulsions.

6. Appareil de mesure de la pression sanguine sur une longue durée selon l'une des revendications 4 ou 5,
**caractérisé en ce que** le moyen (3) pour activer une mesure de la pression sanguine est conçu pour délivrer un signal d'activation lors de la détection d'un temps de parcours des ondulations à impulsions raccourci ou prolongé par rapport à des valeurs déjà déterminées.

7. Appareil de mesure de la pression sanguine sur une longue durée selon l'une des revendications précédentes,
**caractérisé en ce que** l'unité d'évaluation (3) est conçue pour déterminer la pression sanguine de diastole par oscillométrie.

8. Appareil de mesure de la pression sanguine sur une longue durée selon l'une des revendications précédentes,
**caractérisé en ce que** le capteur de mesure (8) pour l'évolution d'un signal ondulatoire à impulsions comprend une source de rayonnement, en particulier une source lumineuse, et un élément récepteur, en particulier une photodiode, et est de préférence applicable sur un doigt ou sur un lobe d'oreille d'une personne.

9. Appareil de mesure de la pression sanguine sur une longue durée selon l'une des revendications précédentes,
**caractérisé en ce que** le capteur de mesure (8) pour l'évolution d'un signal ondulatoire à impulsions est conçu pour déterminer l'évolution de la pression partielle d'oxygène (pO₂) d'une personne, et **en ce que** la mémoire de signaux (2) est conçue pour mémoriser en supplément cette évolution.

10. Appareil de mesure de la pression sanguine sur une longue durée selon l'une des revendications précédentes,
**caractérisé en ce que** l'unité d'évaluation (3) est conçue pour la détection d'irrégularités et de l'évolution du signal ondulatoire à impulsions, en particulier pour la détection de modifications des formes du signal ondulatoire à impulsions ou d'une modification de l'évolution temporelle du signal ondulatoire à impulsions ou encore d'une chute d'amplitude du signal ondulatoire à impulsions par rapport à des valeurs déjà déterminées, et pour évaluer l'évolution des signaux électriques corporels dans la section temporelle dans laquelle une irrégularité a été constatée.

11. Appareil de mesure de la pression sanguine sur une longue durée selon l'une des revendications précédentes,
**caractérisé en ce que** dans l'unité d'évaluation (3) il est prévu un dispositif pour faire la moyenne de l'évolution du signal ondulatoire à impulsions, dispositif qui constate respectivement le début d'une impulsion individuelle à prendre en compte pour une moyenne en raison de la référence temporelle vis-à-vis des signaux électriques corporels respectivement associés.

12. Appareil de mesure de la pression sanguine sur une longue durée selon l'une des revendications 3 à 6,
**caractérisé en ce que** l'unité d'évaluation (3) est conçu pour la détection de signaux d'un stimulateur cardiaque, en particulier de pointes d'amplitude non naturelles, dans les signaux électriques corporels, et pour l'évaluation de l'évolution du signal ondulatoire à impulsions dans la section temporelle dans laquelle des signaux de stimulateur cardiaque ont été constatés.

13. Appareil de mesure de la pression sanguine sur une longue durée selon l'une des revendications précédentes,
**caractérisé en ce qu'**il est réalisé sous forme d'appareil mobile avec un boîtier (1), équipé d'une source d'énergie, susceptible d'être porté sur le corps d'une personne, et/ou
**en ce que** l'unité d'évaluation (3) est prévue dans le boîtier (1) ou réalisée sous forme d'unité externe.

14. Appareil de la mesure de la pression sanguine sur une longue durée selon l'une des revendications 2 à 13,
**caractérisé en ce que** la corrélation entre les valeurs de pression sanguine mesurée et les écarts temporels t₁ existants à l'instant de mesure respectif selon la revendication 2 est déterminé, et lors d'un dépassement d'une valeur limite prédéterminée par la valeur de corrélation déterminée, d'autres valeurs de pression sanguine sont calculées à partir des écarts temporels t₁ détectés entre les mesures de pression sanguine selon la revendication 2.
